# EUROPEAN PATENT APPLICATION

(11) **EP 2 752 477 A1**
(43) Date of publication of application: **09.07.2014**
(21) Application number: 11871427.8
(22) Date of filing: 01.09.2011
(51) Int. Cl.: C10J 3/00, C10G 35/09, B01J 23/42, C10L 1/04

(54) **METHOD FOR OBTAINING BRANCHED ALKANES AND AROMATIC HYDROCARBONS**

(71) Applicant: Guradoor S.L., 38390 Santa Ursula (Tenerife) (ES)
(72) Inventor: GONZÁLEZ GONZÁLEZ, Daniel, 38390 Santa Úrsula (Tenerife) (ES)
(74) Representative: Gil-Vega, Victor
(86) International application number: PCT/ES2011/070616
(87) International publication number: WO 2013/030414

(57) **Abstract**

A method for the recovery of energy from synthesis gas waste products obtained from wet crushed coal, according to a gasification-pyrolysis process, which comprises the submission of the waste products obtained during the production of the synthesis gas to a subsequent treatment, to transform such products into other products of the branched-chain alkane type and aromatic compounds, recovering the hydrogen obtained during these reactions, which will be available to be used at other chemical processing plants, as fuel or simply to be fed back to the gasification-pyrolysis process itself, to enrich the synthesis gas obtained.

## Description

The present invention generally relates to a method for the recovery of energy from the waste products originated in a process aimed at the obtaining of synthesis gas, essentially consisting of a mixture of CO, CO₂ and H₂, and derived from wet crushed coal.

Specifically, the invention refers to a method for the recovery of energy, basically as saturated branched-chain cyclic and alicyclic hydrocarbons, as well as aromatic compounds, from the waste produced in a process for the obtaining of synthesis gas, especially from a gasification-pyrolysis process applied to the wet crushed coal, wherein said residues essentially consist of gases of different compositions, that can be subsequently used as fuels, in some cases, and as raw materials, in other cases.

The reactions involved in the coal gasification process are essentially the following ones:
- C + ½ O₂ →CO ΔHr = -9,25 MJ/Kg
- C + CO₂ →2CO ΔHr = 14,37 MJ/Kg
- C + H₂O →CO + H₂ ΔHr = 10,94 MJ/Kg
- CH₄ + ½ O₂ →CO + 2H₂ ΔHr = -35,7 MJ/kmol
- H₂ + ½ O₂ →H₂O ΔHr = -242 MJ/kmol
- CH₄+ H₂O →CO + 3H₂ ΔHr = 206 MJ/kmol
- CO + H₂O →CO₂ + H₂ ΔHr = -41,1 MJ/kmol

And to a lesser extent and, as secondary reactions,
- CO + ½ O₂ →CO₂ + 67Kcal/mol
- CO + 3H₂→ CH₄ + H₂O + 49 Kcal/mol
- CO₂ + 4H₂→ CH₄ + 2 H₂O + 42 Kcal/mol

As the above-mentioned reactions are actually equilibriums, certain factors, such as temperature or pressure, may significantly alter the output of the gasification process and the nature of the waste products obtained, and basically, the gas obtained has a low percentage of methane, tars (paraffins and olefins) and oils.

As in the case of the processes for the reforming of oil-based products, which are sufficiently known, the exploitation of the aforementioned by-products may involve a reforming method, which can be implemented to increase their volatility (decrease in the molecular size) or to convert linear paraffins into isoparaffins, or olefins and aromatic products and/or naphtenes (cicloalkanes) into aromatic ones. The nature of the final product is influenced by the structure and composition of the naphtha fed, as a result of the nature of the residual mixture of hydrocarbons obtained.

Thus, during the thermal reforming, the reactions are similar to those appeared during the fuel oil cracking: the molecular size is decreased while olefins and certain aromatic compounds are synthesised. For instance, an alkane may be converted into another alkane with less molecular weight plus an olefin, according to the following reaction, where n > x + y, 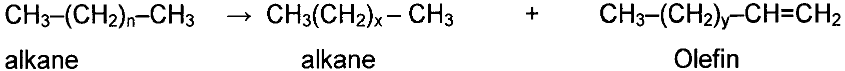 or an alkane may be converted into a cycloalkane, which in turn is converted into an aromatic compound, as it is the case of the reaction below:

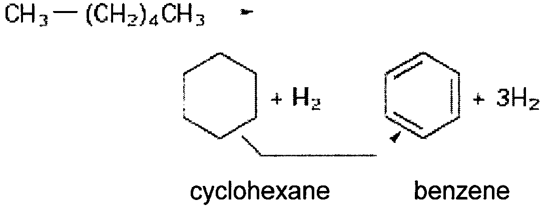

In the case of catalytic reforming, the number of carbon atoms of the constituents of the load remains unchanged. For instance, cyclohexane is transformed into benzene. However, the process is somewhat more complicated. It is possible to convert substituted cyclohexanes into substituted benzenes; linear paraffins, like n-heptane become toluene, and the substituted cyclopentanes may also experience an expansion in the ring and become aromatic compounds. When heavy naphthas are used as by-products, methylnaphthalenes are obtained. Catalytic reforming is a reaction which occurs through carbocations; however, those reactions where aromatic compounds are produced are favoured.

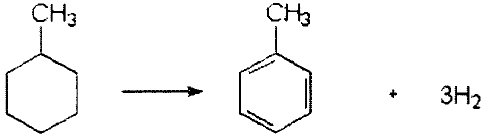

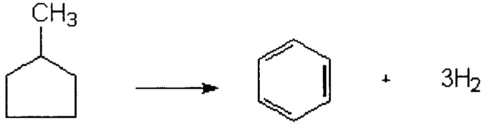

On the other hand, in the case of isomerisation processes, alkanes are converted into their branched-chain isomers. For instance, butane is isomerised to isobutane to be subsequently used in the alkylation of isobutylene and other olefins. The fraction with 5 and 6 carbon atoms, which is naturally found in petrol, is isomerized to obtain products with a high-octane rating that will be subsequently mixed with petrols containing a low-octane rating. For instance,

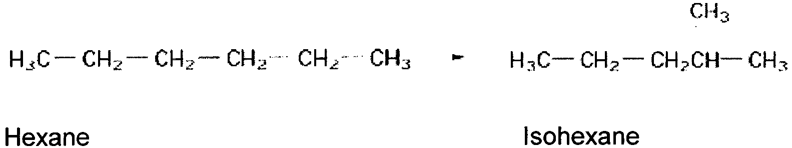

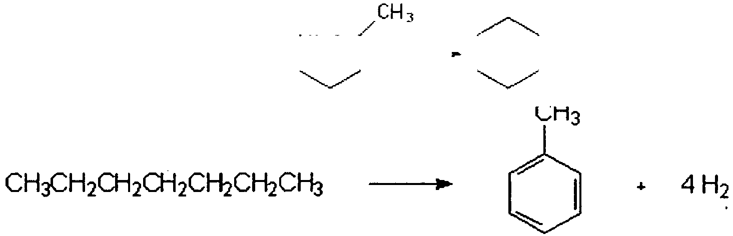

Thus, the object of the invention is to provide a method for energy recovery, basically as saturated cyclic and alicyclic branched-chain hydrocarbons, but also as aromatic compounds, from the waste originated by a method aimed at obtaining synthesis gas, especially from a method of gasification-pyrolysis of wet crushed coal, wherein such residues essentially consist of gases of different compositions, that can be subsequently used as fuels, in some cases, and as raw materials, in other cases.

Different gasoline reforming processes are widely known in the art. Such processes increase the performance of the gasolines and their octane rating. The original content of gasoline obtained from oil is not sufficient to cover the high market demand and consequently, it is necessary to transform some less valuable oil fractions into gasoline. Those molecules to which a higher octane rating is assigned are: branched-chain and aromatic alkanes. The octane rating of linear alkanes and naphthenes is lower; therefore, it is advisable to transform them into isomers (of the branched-chain type) and aromatic compounds, respectively. Linear molecules have shown a trend to detonate under pressure. Therefore, the objective is to "reform" such linear molecules, converting them into branched-chain and cyclic ones. As they are more compact, they do not detonate as a result of pressure. The reforming process can be performed in two different ways, namely through heating (which is called thermal reforming) or through heating, with the assistance of a catalyser (catalytic reforming). Hydrogen is released during the reforming process.

The method herein described allows to recover energy from the waste originated by a method aimed at obtaining synthesis gas, especially from a method of gasification-pyrolysis of wet crushed coal, wherein at least a part of that waste is transformed into compounds with a higher octane-rating, such as branched-chain alkanes and aromatic compounds. As it has been previously mentioned, since hydrogen is released as the final by-product of the process, such hydrogen may be recycled so that it may be subsequently used at chemical plants or fed back to the gasification-pyrolysis process, to enrich the synthesis gas thus obtained.

To that effect, in the method according to the invention, the waste derived from the obtaining of synthesis gas from crushed wet coal, according to a gasification-pyrolysis process, is subject to a subsequent treatment to transform such by-products into different ones, of the branched-chain alkane type and aromatic compounds, thus recovering the hydrogen obtained during such reactions, which will remain available to be used as fuel in other chemical processing plants, but which can also be fed back to the gasification-pyrolysis process, to enrich the synthesis gas thus obtained.

To that effect, downstream of the gasification-pyrolysis reactor, a catalytic reactor is arranged, which operates at a temperature of 1,100°C, which is obtained by means of the energy obtained from the gasification-pyrolysis reactor itself, either as heat or as steam, which has previously been used to feed the gasification-pyrolysis reactor.

Inside the aforementioned catalytic reactor, the reforming and isomerization reactions to obtain aromatic compounds and branched-chain alkanes with a high energy content, are catalysed by means of a platinum-rhenium-alumina-based catalyst, and preferably, a platinum-based catalyst, of the alumina-supported type, or non-supported. The temperature at which the process is carried out allows the development of the above-mentioned chemical reactions, but does not involve the deposition of solid carbon on the surface of the catalyst, since the hydrogen obtained as the by-product of the reaction is essentially eliminated at the catalytic reactor and recycled for other uses or it may be used in the gasification-pyrolysis process itself, as it has been previously mentioned, which avoids the need to carry out a clean-up of the catalysts for its use on an on-going basis, thus facilitating the production of compounds with a high energy content as a subsequent stage to the coal gasification-pyrolysis.

## Claims

1. Method for the recovery of energy from the waste products obtained from the synthesis gas derived from wet crushed coal, according to a gasification-pyrolysis process, **characterised in that** the method comprises the submission of the waste products obtained during the production of the synthesis gas to a subsequent treatment, to transform such products into other products of the branched-chain alkane type and aromatic compounds, recovering the hydrogen obtained during these reactions, which will be available to be used at other chemical processing plants, as fuel or simply to be fed back to the gasification-pyrolysis process itself, to enrich the synthesis gas obtained.

2. Method for the recovery of energy from the synthesis gas waste products according to claim 1, **characterized in that** the reactions used to obtain the aromatic compounds and the branched-chain type alkanes are catalysed by means of a platinum-rhenium-alumina-based catalyst, and preferably, a platinum-based catalyst, of the alumina-supported type, or non-supported.

3. Method for the recovery of energy from the synthesis gas waste products according to claim 1, **characterized in that** the reactions used to obtain the aromatic compounds and the branched-chain type alkanes are carried out inside a catalytic reactor which operates at a temperature of 1100°C and which is located downstream of the gasification-pyrolysis reactor.

4. Method for the recovery of energy from the synthesis gas waste products according to claim 3, **characterised in that** the said temperature is reached by feeding the energy obtained from the gasification-pyrolysis reactor itself, either as heat or as the steam previously used to feed the gasification-pyrolysis reactor.
